# EUROPEAN PATENT SPECIFICATION

(11) **EP 4 221 593 B1**
(45) Date of publication and mention of the grant of the patent: **01.07.2026**
(21) Application number: 21801303.5
(22) Date of filing: 29.09.2021
(51) Int. Cl.: A61B 5/346, A61B 5/367, A61B 5/339, A61B 5/00

(54) **ELECTROPHYSIOLOGY SYSTEM AND METHOD FOR ASSESSING MICRO-REENTRY SITES**
ELEKTROPHYSIOLOGISCHES SYSTEM UND VERFAHREN ZUR BEURTEILUNG VON MIKROWIEDEREINTRITTSSTELLEN
SYSTÈME ET PROCÉDÉ D'ÉLECTROPHYSIOLOGIE POUR ÉVALUER DES SITES DE MICRO-RENTRÉE

(30) Priority: 30.09.2020 US 202063085683 P
(43) Date of publication of application: 09.08.2023
(73) Proprietor: Boston Scientific Scimed Inc., Maple Grove, Minnesota 55311 (US)
(72) Inventor: BENNETT, Nathan H., Cambridge, Massachusetts 02138 (US); STEWART, Brian, Reading, Massachusetts 01864 (US)
(74) Representative: Pfenning, Meinig & Partner mbB
(86) International application number: PCT/US2021/052589
(87) International publication number: WO 2022/072455

(56) References cited:
- US-A1- 2012 184 863
- US-A1- 2018 296 108
- US-A1- 2018 325 401

## Description

### CROSS REFERENCE TO RELATED APPLICATION

This application claims priority to Provisional Application No. 63/085,683, filed September 30, 2020.

### TECHNICAL FIELD

The present disclosure relates to electrophysiology systems and methods for processing cardiac electrical signals.

### BACKGROUND

Use of minimally invasive procedures, such as catheter ablation, to treat a variety of heart conditions, such as supraventricular and ventricular arrhythmias, is becoming increasingly more prevalent. Such procedures involve the mapping of electrical activity in the heart (e.g., based on cardiac signals), such as at various locations on the endocardium surface ("cardiac mapping"), to identify the site of origin of the arrhythmia followed by a targeted ablation of the site. To perform such cardiac mapping, a catheter with one or more electrodes can be inserted into the patient's cardiac chamber.

Conventional three-dimensional (3D) mapping techniques include contact mapping, non-contact mapping, and a combination of contact and non-contact mapping. In both contact and non-contact mapping, one or more catheters are advanced into the heart. With some catheters, once in the chamber, the catheter may be deployed to assume a 3D shape. In contact mapping, physiological signals resulting from the electrical activity of the heart are acquired with one or more electrodes located at the catheter distal tip after determining that the tip is in stable and steady contact with the endocardium surface of a particular cardiac chamber. In non-contact-based mapping systems, using the signals detected by the non-contact electrodes and information on chamber anatomy and relative electrode location, the system provides physiological information regarding the endocardium of the cardiac chamber. Location and electrical activity are usually measured sequentially on a point-by-point basis at about 50 to 200 points on the internal surface of the heart to construct an electro-anatomical depiction of the heart. The generated map may then serve as the basis for deciding on a therapeutic course of action, for example, tissue ablation, to alter the propagation of the heart's electrical activity and to restore normal heart rhythm.

In many conventional mapping systems, the clinician visually inspects or examines the captured electrograms (EGMs), which increases examination time and cost. During an automatic electro-anatomical mapping process, however, approximately 6,000 to 20,000 intracardiac electrograms (EGMs) may be captured, which does not lend itself to being manually inspected in full by a clinician (e.g., a physician) for a diagnostic assessment, EGM categorization, and/or the like. Typically mapping systems extract scalar values from each EGM to construct voltage, activation, or other map types to depict overall patterns of activity within the heart. While maps reduce the need to inspect the captured EGMs, they also condense the often complex and useful information in the EGMs. Further, maps may be misleading due to electrical artifacts or inappropriate selection of features such as activation times. Additionally, due to the complex nature of conventional techniques, cardiac maps often are not suitable for accurate and efficient interpretation.

Cardiac mapping may also be used to detect atrial tachycardia sites. Atrial tachycardia (AT) is a type of abnormal heart rhythm, or arrhythmia. It occurs when the electrical signal that controls the heartbeat starts from an unusual location in the atria with rapid repeats, causing the atria to beat too quickly. Atrial tachycardias may be classified into three broad categories: focal ATs, macro-reentry ATs and micro-reentry ATs. Focal ATs may occur in structurally normal hearts but can also occur in patients with heart disease. Macro-reentry ATs may occur in the setting of atrial fibrosis. Micro-reentry ATs may occur in the setting of diseased atrial myocardium that supports very slow conduction.

Document US 2018/296108 A1 describes systems and methods for facilitating processing of cardiac information based on sensed electrical signals. The systems include a processing unit configured to receive a set of electrical signals; receive an indication of a measurement location corresponding to each electrical signal of the set of electrical signals; and generate, based on at least one of an annotation waveform corresponding to each electrical signal of the set of electrical signals and a set of annotation mapping values, an annotation histogram.

Document US 2018/325401 A1 describes a system for facilitating display of cardiac information. The system includes a display device configured to present a cardiac map; and a processing unit configured to: receive electrical signals and indications of measurement locations corresponding to the electrical signals; generate, based on the electrical signals, the cardiac map, which includes annotations representing cardiac signal features; and determine a set of interesting cardiac signal features. The processing unit also may determine, based on the set of interesting cardiac signal features, a region of interest; and facilitate display, via the display device, of the cardiac map and a representation of the region of interest. The representation of the region of interest includes a first display parameter value that is different from a second display parameter value, where the second display parameter value is associated with at least one cardiac signal feature that is not included within the region of interest.

Document US 2012/184863 A1 describes a method for providing information about a patient's heart. The method comprises measuring signals from one or more electrodes at multiple positions in the heart cavity in response to electrical activity in the patient's heart cavity over multiple heart beat cycles; generating, by a computer, annotation information for the measured signals by applying one or more operators to the measured signals to identify at least one of regions of the heart having double deflections, regions of the heart having multiple deflections, regions of the heart having fractionation, regions of the heart having double activation, and regions of the heart having no activation; and generating, by the computer, an electroanatomical representation of the patient's heart that includes at least some of the annotation information.

### SUMMARY

The invention is defined in the independent claims. As recited in examples, Example 1 is a system for processing cardiac information. The system comprises a processing unit configured to: receive one or more cardiac electrical signals from one or more electrodes disposed within a cardiac
chamber, wherein the cardiac electrical signals are acquired over a cardiac beat; receive an indication of a measurement location corresponding to each of the cardiac electrical signals; analyze the one or more cardiac electrical signals to calculate a plurality of duty cycle values, each calculated duty cycle value corresponding to a respective one of a plurality of pre-selected cycle length windows; calculate an average duty cycle of the calculated duty cycle values over the plurality of pre-selected cycle length windows; and facilitate presentation, on a display device, a three-dimensional electroanatomical map overlayed with an annotation representing the calculated average duty cycle based on the measurement locations for the cardiac electrical signals associated therewith.

Example 2 is the system of Example 1, wherein the cardiac electrical signals comprise intra-cardiac electrograms (EGMs).

Example 3 is the system of Example 2, wherein the processing unit is configured to calculate the plurality of duty cycle values by determining an activation duration associated with the cardiac beat and dividing the activation duration by each of the plurality of pre-selected cycle length windows.

Example 4 is the system of Example 2, wherein the analysis of the one or more cardiac electrical signals includes generating an activation waveform therefrom, the activation waveform being based on deflections of the one or more analyzed cardiac electrical signals from a signal baseline.

Example 5 is the system of Example 4, wherein the processing unit is further configured to determine the activation duration based on the activation waveform.

Example 6 is the system of either of Examples 4 or 5, wherein the activation waveform comprises values indicative of probabilities that the deflections represent activations of cardiac tissue.

Example 7 is the system of any of Examples 4-6, wherein the processing unit is configured to calculate the plurality of duty cycle values by calculating an average activation waveform value over each of the plurality of pre-selected cycle length windows.

Example 8 is the system of any of Examples 1-7, wherein the calculated average duty cycle represents a probability that cardiac tissue corresponding to the measurement locations define a micro-reentry site.

Example 9 is the system of any of Examples 1-8, wherein the plurality of pre-selected cycle length windows fall within a range of from 140 milliseconds to 2000 milliseconds.

Example 10 is the system of any of Examples 1-9, further comprising a display device operatively connected to the processing unit and configured to display the three-dimensional anatomical map overlayed with the annotations.

Example 11 is the system of any of Examples 1-10, wherein the processing unit is configured to calculate the average duty cycle value for each cardiac electrical signal.

Example 12 is the system of any of Examples 1-10, wherein the processing unit is configured to aggregate a plurality of cardiac electrical signals having associated measurement locations within a specified region, and to calculate the average duty cycle value for the aggregated cardiac electrical signals.

Example 13 is a method of processing cardiac information. The method comprising: receiving one or more cardiac electrical signals acquired over a cardiac beat; analyzing the one or more cardiac electrical signals and calculating a plurality of duty cycle values, each calculated duty cycle value corresponding to a respective one of a plurality of pre-selected cycle length windows; calculating an average of the calculated duty cycle values over the plurality of pre-selected cycle length windows; and displaying, on a display device, a three-dimensional anatomical map overlayed with an annotation representing the calculated average duty cycle.

Example 14 is the method of Example 13, wherein the analyzing the one or more cardiac electrical signals includes generating an activation waveform therefrom, the activation waveform being based on deflections of the one or more analyzed cardiac electrical signals from a signal baseline.

Example 15 is the method of Example 13 or 14, wherein calculating the plurality of duty cycle values includes calculating the plurality of duty cycle values based on the activation waveform and each of the plurality of pre-selected cycle length windows.

Example 16 is a system for processing cardiac information. The system comprises a processing unit configured to: receive one or more cardiac electrical signals from one or more electrodes disposed within a cardiac chamber, wherein the cardiac electrical signals are acquired over a cardiac beat; receive an indication of a measurement location corresponding to each of the cardiac electrical signals; analyze the one or more cardiac electrical signals to calculate a plurality of duty cycle values, each calculated duty cycle value corresponding to a respective one of a plurality of pre-selected cycle length windows; calculate an average duty cycle of the calculated duty cycle values over the plurality of pre-selected cycle length windows; and facilitate presentation, on a display device, a three-dimensional electroanatomical map overlayed with an annotation representing the calculated average duty cycle based on the measurement locations for the cardiac electrical signals associated therewith.

Example 17 is the system of Example 16, wherein the cardiac electrical signals comprise intra-cardiac electrograms (EGMs).

Example 18 is the system of Example 17, wherein the processing unit is configured to calculate the plurality of duty cycle values by determining an activation duration associated with the cardiac beat and dividing the activation duration by each of the plurality of pre-selected cycle length windows.

Example 19 is the system of Example 17, wherein the analysis of the one or more cardiac electrical signals includes generating an activation waveform therefrom, the activation waveform being based on deflections of the one or more analyzed cardiac electrical signals from a signal baseline.

Example 20 is the system of Example 19, wherein the processing unit is further configured to determine the activation duration based on the activation waveform.

Example 21 is the system of Example 19, wherein the activation waveform comprises values indicative of probabilities that the deflections represent activations of cardiac tissue.

Example 22 is the system of Example 19, wherein the processing unit is configured to calculate the plurality of duty cycle values by calculating an average activation waveform value over each of the plurality of pre-selected cycle length windows.

Example 23 is the system of Example 16, wherein the calculated average duty cycle represents a probability that cardiac tissue corresponding to the measurement locations define a micro-reentry site.

Example 24 is the system of Example 16, wherein the plurality of pre-selected cycle length windows fall within a range of from 140 milliseconds to 2000 milliseconds.

Example 25 is the system of Example 16, further comprising a display device operatively connected to the processing unit and configured to display the three-dimensional anatomical map overlayed with the annotations.

Example 26 is the system of Example 16, wherein the processing unit is configured to calculate the average duty cycle value for each cardiac electrical signal.

Example 27 is the system of Example 16, wherein the processing unit is configured to aggregate a plurality of cardiac electrical signals having associated measurement locations within a specified region, and to calculate the average duty cycle value for the aggregated cardiac electrical signals.

Example 28 is a method of processing cardiac information. The method comprising: receiving one or more cardiac electrical signals acquired over a cardiac beat; analyzing the one or more cardiac electrical signals and calculating a plurality of duty cycle values, each calculated duty cycle value corresponding to a respective one of a plurality of pre-selected cycle length windows; calculating an average of the calculated duty cycle values over the plurality of pre-selected cycle length windows; and displaying, on a display device, a three-dimensional anatomical map overlayed with an annotation representing the calculated average duty cycle.

Example 29 is the method of Example 28, wherein the analyzing the one or more cardiac electrical signals includes generating an activation waveform therefrom, the activation waveform being based on deflections of the one or more analyzed cardiac electrical signals from a signal baseline.

Example 30 is the method of Example 28, wherein the calculating the plurality of duty cycle values includes calculating the plurality of duty cycle values based on the activation waveform and each of the plurality of pre-selected cycle length windows.

Example 31 is the method of Example 28, wherein the cardiac electrical signals comprise intra-cardiac electrograms (EGMs).

Example 32 is the method of Example 29, wherein the activation waveform comprises values indicative of probabilities that the deflections represent activations of cardiac tissue.

Example 33 is the method of Example 29, wherein the calculating the plurality of duty cycle values includes calculating an average activation waveform value over each of the plurality of pre-selected cycle length windows.

Example 34 is the method of Example 28, wherein the calculated average duty cycle represents a probability that cardiac tissue corresponding to the measurement locations define a micro-reentry site.

Example 35 is the method of Example 28, wherein the calculating the plurality of duty cycle values includes aggregating a plurality of cardiac electrical signals having associated measurement locations within a specified region and calculating the average duty cycle value for the aggregated cardiac electrical signals.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 shows a schematic diagram of an exemplary embodiment of an electrophysiology system, in accordance with embodiments of the subject matter disclosed herein.
FIG. 2 is a block diagram of an illustrative processing unit, in accordance with embodiments of the present disclosure.
FIG. 3 is a flow diagram of an illustrative process for automated anatomical mapping, in accordance with embodiments of the present disclosure.
FIG. 4A is an example flow diagram depicting an illustrative method of processing cardiac electrical signals and generated activation waveform, in accordance with some embodiments of the present disclosure.
FIG. 4B is an example flow diagram depicting an illustrative method 400B of processing cardiac electrical signals and generated activation waveform, in accordance with some embodiments of the present disclosure.
FIG. 5A depicts an exemplary graphical representation illustrating electrical signals received from a mapping catheter.
FIG. 5B depicts a waveform of a raw cardiac electrical signal and an activation waveform corresponding to the cardiac electrical signal.
FIG. 6 depicts an illustrative example of an anatomical map with annotations representing average duty cycle values.

While the invention is amenable to various modifications and alternative forms, specific embodiments have been shown by way of example in the drawings and are described in detail below. The intention, however, is not to limit the invention to the particular embodiments described. The scope of the invention is defined by the appended claims.

### DETAILED DESCRIPTION

As the terms are used herein with respect to measurements (e.g., dimensions, characteristics, attributes, components, etc.), and ranges thereof, of tangible things (e.g., products, inventory, etc.) and/or intangible things (e.g., data, electronic representations of currency, accounts, information, portions of things (e.g., percentages, fractions), calculations, data models, dynamic system models, algorithms, parameters, etc.), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error; differences in measurement and/or manufacturing equipment calibration; human error in reading and/or setting measurements; adjustments made to optimize performance and/or structural parameters in view of other measurements (e.g., measurements associated with other things); particular implementation scenarios; imprecise adjustment and/or manipulation of things, settings, and/or measurements by a person, a computing device, and/or a machine; system tolerances; control loops; machine-learning; foreseeable variations (e.g., statistically insignificant variations, chaotic variations, system and/or model instabilities, etc.); preferences; and/or the like.

Although illustrative methods may be represented by one or more drawings (e.g., flow diagrams, communication flows, etc.), the drawings should not be interpreted as implying any requirement of, or particular order among or between, various steps disclosed herein. However, certain some embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

As used herein, the term "based on" is not meant to be restrictive, but rather indicates that a determination, identification, prediction, calculation, and/or the like, is performed by using, at least, the term following "based on" as an input. For example, predicting an outcome based on a particular piece of information may additionally, or alternatively, base the same determination on another piece of information.

Identification of micro-reentry atrial tachycardias (AT) in cardiac maps (e.g., activation maps) is difficult. Even if a micro-reentry is suspected, tracking down the location of the micro-reentry can be difficult and time consuming. Additionally, a slow micro-reentry AT may look similar to a focal AT but with a small area of mixed timing at the center. In many cases, an electroanatomical map of micro-reentry AT contains many areas of disorganization and dissociation, giving the map an atrial fibrillation (AF) like appearance. Embodiments of the present disclosure facilitate the assessments of micro-reentry AT sites. In some embodiments, certain electrogram characteristics (e.g., average duty cycle values over a plurality of cycle length windows) are generated and evaluated to determine probabilities of micro-reentry sites. In some embodiments, an anatomical map with annotations of electrogram characteristics is presented to facilitate the assessments of micro-reentry sites. In some embodiments, activation waveforms and activation waveform values are used in the assessment.

An activation waveform, or referred to as an annotation waveform, is a set of activation waveform values and may include, for example, a set of discrete activation waveform values (e.g., a set of activation waveform values, a set of activation time annotations, etc.), a function defining an activation waveform curve, and/or the like. In some embodiments, each data point of an activation waveform represents the per-sample "probability" of tissue activation. In some embodiments, the activation waveform may be displayed, used to present in an activation propagation map, used to facilitate diagnoses, used to facilitate classification of electrical signals, and/or the like. To perform aspects of embodiments of the methods described herein, the cardiac electrical signals may be obtained from a mapping catheter (e.g., associated with a mapping system), which may be used in conjunction with other equipment typically used in an electrophysiology lab, for example, a recording system, a coronary sinus (CS) catheter or other reference catheter, an ablation catheter, a memory device (e.g., a local memory, a cloud server, etc.), a communication component, a medical device (e.g., an implantable medical device, an external medical device, a telemetry device, etc.), and/or the like.

As the term is used herein, a sensed cardiac electrical signal may refer to one or more sensed signals. Each cardiac electrical signal may include a number of intracardiac electrograms (EGMs) sensed within a cardiac chamber and may include any number of features that may be ascertained by aspects of an electrophysiology system. Examples of cardiac electrical signal features include, but are not limited to, activation times, activations, activation waveforms, filtered activation waveforms, minimum voltage values, maximum voltages values, maximum negative time-derivatives of voltages, instantaneous potentials, voltage amplitudes, dominant frequencies, peak-to-peak voltages, and/or the like. A cardiac electrical signal feature may refer to one or more features extracted from one or more cardiac electrical signals, derived from one or more features that are extracted from one or more cardiac electrical signals, and/or the like. Additionally, a representation, on a cardiac and/or a surface map, of a cardiac electrical signal feature may represent one or more cardiac electrical signal features, an interpolation of a number of cardiac electrical signal features, and/or the like.

Each cardiac signal also may be associated with a set of respective position coordinates that corresponds to the location at which the cardiac electrical signal was sensed. Each of the respective position coordinates for the sensed cardiac signals may include three-dimensional Cartesian coordinates, polar coordinates, and/or the like. In some cases, other coordinate systems can be used. In some embodiments, an arbitrary origin is used and the respective position coordinates refer to positions in space relative to the arbitrary origin. Since, in some embodiments, the cardiac signals may be sensed on the cardiac surfaces, the respective position coordinates may be on the endocardial surface, epicardial surface, in the mid-myocardium of the patient's heart, and/or in the vicinity of one of one of these.

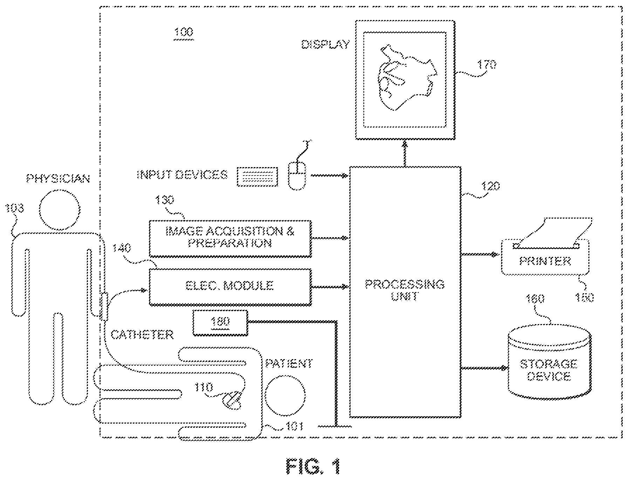

FIG. 1 shows a schematic diagram of an exemplary embodiment of an electrophysiology system 100. As indicated above, embodiments of the subject matter disclosed herein may be implemented in a mapping system (e.g., a cardiac mapping system), while other embodiments may be implemented in an ablation system, a recording system, a computer analysis system, and/or the like. The electrophysiology system 100 includes a moveable catheter 110 having multiple spatially distributed electrodes. During a signal-acquisition stage, the catheter 110 is displaced to multiple locations within the cardiac chamber into which the catheter 110 is inserted. In some embodiments the distal end of the catheter 110 is fitted with multiple electrodes spread somewhat uniformly over the catheter. For example, the electrodes may be mounted on the catheter 110 following a 3D olive shape, a basket shape, and/or the like. The electrodes are mounted on a device capable of deploying the electrodes into the desired shape while inside the heart, and retracting the electrodes when the catheter is removed from the heart. To allow deployment into a 3D shape in the heart, electrodes may be mounted on a balloon, shape memory material such as Nitinol, actuable hinged structure, and/or the like. According to embodiments, the catheter 110 may be a mapping catheter, an ablation catheter, a diagnostic catheter, a CS catheter, and/or the like. For example, aspects of embodiments of the catheter 110, the electrical signals obtained using the catheter 110, and subsequent processing of the electrical signals, as described herein, may also be applicable in implementations having a recording system, ablation system, and/or any other system having a catheter with electrodes that may be configured to obtain cardiac electrical signals.

At each of the locations to which the catheter 110 is moved, the catheter's multiple electrodes acquire signals resulting from the electrical activity in the heart. Consequently, reconstructing and presenting to a user (such as a doctor and/or technician) physiological data pertaining to the heart's electrical activity may be based on information acquired at multiple locations, thereby providing a more accurate and faithful reconstruction of physiological behavior of the endocardium surface. The acquisition of signals at multiple catheter locations in the cardiac chamber enables the catheter to effectively act as a "mega-catheter" whose effective number of electrodes and electrode span is proportional to the product of the number of locations in which signal acquisition is performed and the number of electrodes the catheter has.

To enhance the quality of the reconstructed physiological information at the endocardium surface, in some embodiments the catheter 110 is moved to more than three locations (for example, more than 5, 10, or even 50 locations) within the cardiac chamber. Further, the spatial range over which the catheter is moved may be larger than one third (1/3) of the diameter of the heart cavity (for example, larger than 35%, 40%, 50% or even 60% of the diameter of the heart cavity). Additionally, in some embodiments the reconstructed physiological information is computed based on signals measured over several heart beats, either at a single catheter location within the cardiac chamber or over several locations. In circumstances where the reconstructed physiological information is based on multiple measurements over several heart beats, the measurements may be synchronized with one another so that the measurement are performed at approximately the same phase of the heart cycle. The signal measurements over multiple beats may be synchronized based on features detected from physiological data such as surface electrocardiograms (ECGs) and/or intracardiac electrograms (EGMs).

The electrophysiology system 100 further includes a processing unit 120 which performs several of the operations pertaining to the assessment, including processing cardiac electrical signals collected from electrodes and/or catheters. The processing unit 120 may perform a mapping procedure, including, for example, the reconstruction procedure to determine the physiological information at the endocardium surface (e.g., as described above) and/or within a cardiac chamber. The processing unit 120 also may perform a catheter registration procedure. The processing unit 120 also may generate a 3D grid used to aggregate the information captured by the catheter 110 and to facilitate display of portions of that information.

The location of the catheter 110 inserted into the cardiac chamber can be determined using a conventional sensing and tracking system 180 that provides the 3D spatial coordinates of the catheter and/or its multiple electrodes with respect to the catheter's coordinate system as established by the sensing and tracking system. These 3D spatial locations may be used in building the 3D grid. Embodiments of the system 100 may use a hybrid location technology that combines impedance location with magnetic location technology. This combination may enable the system 100 to accurately track catheters that are connected to the system 100. Magnetic location technology uses magnetic fields generated by a localization generator positioned under the patient table to track catheters with magnetic sensors. Impedance location technology may be used to track catheters that may not be equipped with a magnetic location sensor, which may be used with surface ECG patches.

In some embodiments, to perform a mapping procedure and reconstruct physiological information on the endocardium surface, the processing unit 120 may align the coordinate system of the catheter 110 with the endocardium surface's coordinate system. The processing unit 120 (or some other processing component of the system 100) may determine a coordinate system transformation function that transforms the 3D spatial coordinates of the catheter's locations into coordinates expressed in terms of the endocardium surface's coordinate system, and/or vice-versa. In some cases, such a transformation may not be necessary, as some embodiments of the 3D grid may be used to capture contact and non-contact EGMs, and select mapping values based on statistical distributions associated with nodes of the 3D grid. The processing unit 120 also may perform post-processing operations on the physiological information to extract and display useful features of the information to the operator of the system 100 and/or other persons (e.g., a physician).

According to embodiments, the signals acquired by the multiple electrodes of catheter 110 are passed to the processing unit 120 via an electrical module 140, which may include, for example, a signal conditioning component. The electrical module 140 receives the signals communicated from the catheter 110 and performs signal enhancement operations on the signals before they are forwarded to the processing unit 120. The electrical module 140 may include signal conditioning hardware, software, and/or firmware that may be used to amplify, filter and/or sample intracardiac potential measured by one or more electrodes. The intracardiac signals typically have a maximum amplitude of 60mV, with a mean of a few millivolts.

In some embodiments, the signals are filtered by a bandpass filter with a frequency range (e.g., 0.5-500Hz) and sampled with analog to digital converters (e.g., with 15-bit resolution at 1kHz). To avoid interference with electrical equipment in the room, the signals may be filtered to remove the frequency corresponding to the power supply (e.g., 60 Hz). Other types of signal processing operations such as spectral equalization, automatic gain control, etc. may also take place. In some implementations, the intracardiac signals may be unipolar signals measured relative to a reference (which may be a virtual reference). In such implementations, the reference can be, for example, a coronary sinus catheter or Wilson's Central Terminal (WCT), from which the signal processing operations may compute differences to generate multipolar signals (e.g., bipolar signals, tripolar signals, etc.). In some other implementations, the signals may be processed (e.g., filtered, sampled, etc.) before and/or after generating the multipolar signals. The resultant processed signals are forwarded by the electrical module 140 to the processing unit 120 for further processing.

As further shown in FIG. 1, the electrophysiology system 100 also may include peripheral devices such as a printer 150 and/or display device 170, both of which may be interconnected to the processing unit 120. Additionally, the electrophysiology system 100 includes storage device 160 that may be used to store data acquired by the various interconnected modules, including the volumetric images, raw data measured by electrodes and/or the resultant endocardium representation computed therefrom, the partially computed transformations used to expedite the mapping procedures, the reconstructed physiological information corresponding to the endocardium surface, and/or the like.

In some embodiments, the processing unit 120 may be configured to automatically improve the accuracy of its algorithms by using one or more artificial intelligence techniques (e.g., machine learning models, deep learning models), classifiers, and/or the like. In some embodiments, for example, the processing unit may use one or more supervised and/or unsupervised techniques such as, for example, support vector machines (SVMs), k-nearest neighbor techniques, neural networks, convolutional neural networks, recurrent neural networks, and/or the like. In some embodiments, classifiers may be trained and/or adapted using feedback information from a user, other metrics, and/or the like.

The illustrative electrophysiology system 100 shown in FIG. 1 is not intended to suggest any limitation as to the scope of use or functionality of embodiments of the present disclosure. Neither should the illustrative electrophysiology system 100 be interpreted as having any dependency or requirement related to any single component or combination of components illustrated therein. Additionally, various components depicted in FIG. 1 may be, in some embodiments, integrated with various ones of the other components depicted therein (and/or components not illustrated), all of which are considered to be within the ambit of the subject matter disclosed herein. For example, the electrical module 140 may be integrated with the processing unit 120. Additionally, or alternatively, aspects of embodiments of the electrophysiology system 100 may be implemented in a computer analysis system configured to receive cardiac electrical signals and/or other information from a memory device (e.g., a cloud server, a mapping system memory, etc.), and perform aspects of embodiments of the methods described herein for processing cardiac information (e.g., determining annotation waveforms, etc.). That is, for example, a computer analysis system may include a processing unit 120, but not a mapping catheter.

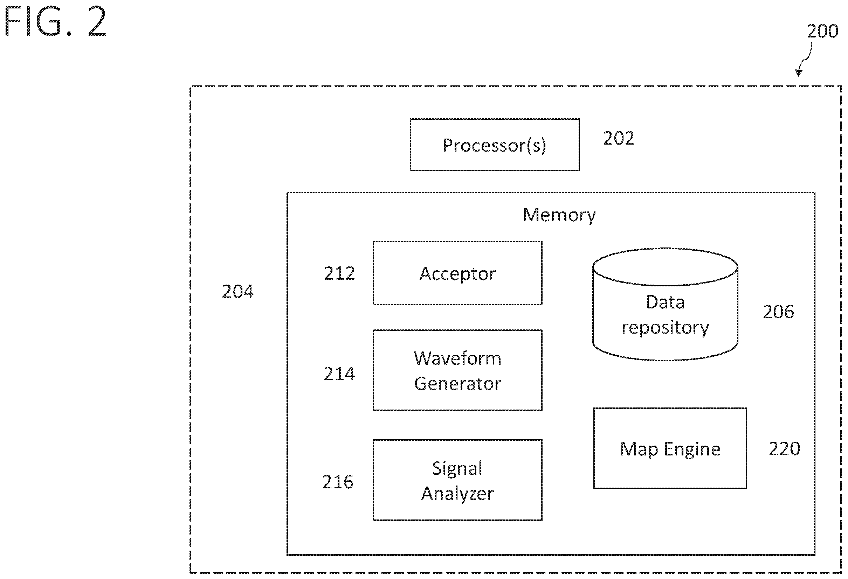

FIG. 2 is a block diagram of an illustrative processing unit 200, in accordance with embodiments of the present disclosure. The processing unit 200 may be, be similar to, include, or be included in the processing unit 120 depicted in FIG. 1. As shown in FIG. 2, the processing unit 200 may be implemented on a computing device that includes one or more processors 202 and one or more memories 204. Although the processing unit 200 is referred to herein in the singular, the processing unit 200 may be implemented in multiple instances (e.g., as a server cluster), distributed across multiple computing devices, instantiated within multiple virtual machines, and/or the like. One or more components of the electrophysiology system may be stored in the memory 204. In some embodiments, the processor 202 may be configured to instantiate the one or more components to generate an activation waveform, a set of signal analysis results, electrogram characteristics, a histogram, and a cardiac map, any one or more of which may be stored in the data repository 206.

As depicted in FIG. 2, the processing unit 200 may include an acceptor 212 configured to receive electrical signals from a mapping catheter (e.g., the mapping catheter 110 depicted in FIG. 1). The measured electrical signals may include a number of intracardiac electrograms (EGMs) sensed within a patient's heart. The acceptor 212 may also receive an indication of a measurement location corresponding to each of the electrical signals. In some embodiments, the acceptor 212 may be configured to determine whether to accept the electrical signals that have been received. The acceptor 212 may utilize any number of different components and/or techniques to determine which electrical signals or beats to accept, such as filtering, beat matching, morphology analysis, positional information (e.g., catheter motion), respiration gating, and/or the like. The received electrical signals and/or the processed electrical signals may be stored in the data repository 206.

In embodiments, the accepted electrical signals are received by an activation waveform generator 214 that is configured to extract at least one activation feature from each of the electrical signals, in cases in which the electrical signal includes an activation feature to extract. In some embodiments, the at least one activation feature includes at least one value corresponding to at least one annotation metric. The at least one feature may include at least one event, where the at least one event includes the at least one value corresponding to the at least one metric and/or at least one corresponding time (a corresponding time does not necessarily exist for each activation feature). In some embodiments, the at least one metric may include, for example, an activation time, minimum voltage value, maximum voltage value, maximum negative time-derivative of voltage, an instantaneous potential, a voltage amplitude, a dominant frequency, a peak-to-peak voltage, an activation duration, and/or the like. In some embodiments, the activation waveform generator 214 may be configured to detect activations and to generate an activation waveform. In some cases, the waveform generator 214 can use any one of activation waveform embodiments, for example, including those described in U.S. Patent Publication 2018/0296113, entitled "ANNOTATION WAVEFORM".

As illustrated in FIG. 2, the processing unit 200 includes a signal analyzer 216 to analyze the received cardiac electrical signals and/or the activation waveform generated by the activation waveform generator 214. In embodiments, the signal analyzer 216 is configured to determine certain characteristics of the received cardiac electrical signals (e.g., average duty cycle values, activation duration, etc.). In embodiments, the signal analyzer may determine micro-reentry probabilities based on the determined characteristics of the cardiac electrical signals. Additionally, the processing unit 200 includes a map engine 220 that is configured to facilitate presentation of a map corresponding to a cardiac surface based on the electrical signals. In some embodiments, the map may include a voltage map, an activation map, a fractionation map, velocity map, confidence map, and/or the like. In some embodiments, the map may include overlaid annotations representing characteristics of the cardiac electrical signals at corresponding measurement locations.

The illustrative processing unit 200 shown in FIG. 2 is not intended to suggest any limitation as to the scope of use or functionality of embodiments of the present disclosure. Neither should the illustrative processing unit 200 be interpreted as having any dependency or requirement related to any single component or combination of components illustrated therein. Additionally, any one or more of the components depicted in FIG. 2 may be, in some embodiments, integrated with various ones of the other components depicted therein (and/or components not illustrated), all of which are considered to be within the ambit of the subject matter disclosed herein. For example, the acceptor 212 may be integrated with the mapping engine 220. In some embodiments, the processing unit 200 may not include an acceptor 212, while in other embodiments, the acceptor 212 may be configured to receive electrical signals from a memory device, a communication component, and/or the like.

Additionally, the processing unit 200 may (alone and/or in combination with other components of the system 100 depicted in FIG. 1, and/or other components not illustrated) perform any number of different functions and/or processes associated with electroanatomical mapping (e.g., triggering, blanking, field mapping, etc.) such as, for example, those described in U.S. Patent Publication 2018/0296113, entitled "ANNOTATION WAVEFORM;" U.S. Patent 8,428,700, entitled "ELECTROANATOMICAL MAPPING;" U.S. Patent 8,948,837, entitled "ELECTROANATOMICAL MAPPING;" U.S. Patent 8,615,287, entitled "CATHETER TRACKING AND ENDOCARDIUM REPRESENTATION GENERATION;" U.S. Patent Publication 2015/0065836, entitled "ESTIMATING THE PREVALENCE OF ACTIVATION PATTERNS IN DATA SEGMENTS DURING ELECTROPHYSIOLOGY MAPPING;" U.S. Patent 6,070,094, entitled "SYSTEMS AND METHODS FOR GUIDING MOVABLE ELECTRODE ELEMENTS WITHIN MULTIPLE-ELECTRODE STRUCTURE;" U.S. Patent 6,233,491, entitled "CARDIAC MAPPING AND ABLATION SYSTEMS;" U.S. Patent 6,735,465, entitled "SYSTEMS AND PROCESSES FOR REFINING A REGISTERED MAP OF A BODY CAVITY".

According to embodiments, various components of the electrophysiology system 100, illustrated in FIG. 1, and/or the processing unit 200, illustrated in FIG. 2, may be implemented on one or more computing devices. A computing device may include any type of computing device suitable for implementing embodiments of the disclosure. Examples of computing devices include specialized computing devices or general-purpose computing devices such "workstations," "servers," "laptops," "desktops," "tablet computers," "hand-held devices," "general-purpose graphics processing units (GPGPUs)," and the like, all of which are contemplated within the scope of FIGS. 1 and 2 with reference to various components of the system 100 and/or processing unit 200.

In some embodiments, a computing device includes a bus that, directly and/or indirectly, couples the following devices: a processor, a memory, an input/output (I/O) port, an I/O component, and a power supply. Any number of additional components, different components, and/or combinations of components may also be included in the computing device. The bus represents what may be one or more busses (such as, for example, an address bus, data bus, or combination thereof). Similarly, in some embodiments, the computing device may include a number of processors, a number of memory components, a number of I/O ports, a number of I/O components, and/or a number of power supplies. Additionally, any number of these components, or combinations thereof, may be distributed and/or duplicated across a number of computing devices.

In some embodiments, memory (e.g., the storage device 160 depicted in FIG. 1, the memory 204 and/or the data repository 206 depicted in FIG. 2) includes computer-readable media in the form of volatile and/or nonvolatile memory, transitory and/or non-transitory storage media and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In some embodiments, the memory 160 and/or 204 stores computer-executable instructions for causing a processor (e.g., the processing unit 120 depicted in FIG. 1 and/or the processor 202 depicted in FIG. 2) to implement aspects of embodiments of system components discussed herein and/or to perform aspects of embodiments of methods and procedures discussed herein.

Computer-executable instructions may include, for example, computer code, machine-useable instructions, and the like such as, for example, program components capable of being executed by one or more processors associated with a computing device. Examples of such program components include the acceptor 212, the waveform generator 214, the signal analyzer 216, and the mapping engine 220. Program components may be programmed using any number of different programming environments, including various languages, development kits, frameworks, and/or the like. Some or all of the functionality contemplated herein may also, or alternatively, be implemented in hardware and/or firmware.

The data repository 206 may be implemented using any one of the configurations described below. A data repository may include random access memories, flat files, XML files, and/or one or more database management systems (DBMS) executing on one or more database servers or a data center. A database management system may be a relational (RDBMS), hierarchical (HDBMS), multidimensional (MDBMS), object oriented (ODBMS or OODBMS) or object relational (ORDBMS) database management system, and the like. The data repository may be, for example, a single relational database. In some cases, the data repository may include a plurality of databases that can exchange and aggregate data by data integration process or software application. In an exemplary embodiment, at least part of the data repository 206 may be hosted in a cloud data center. In some cases, a data repository may be hosted on a single computer, a server, a storage device, a cloud server, or the like. In some other cases, a data repository may be hosted on a series of networked computers, servers, or devices. In some cases, a data repository may be hosted on tiers of data storage devices including local, regional, and central.

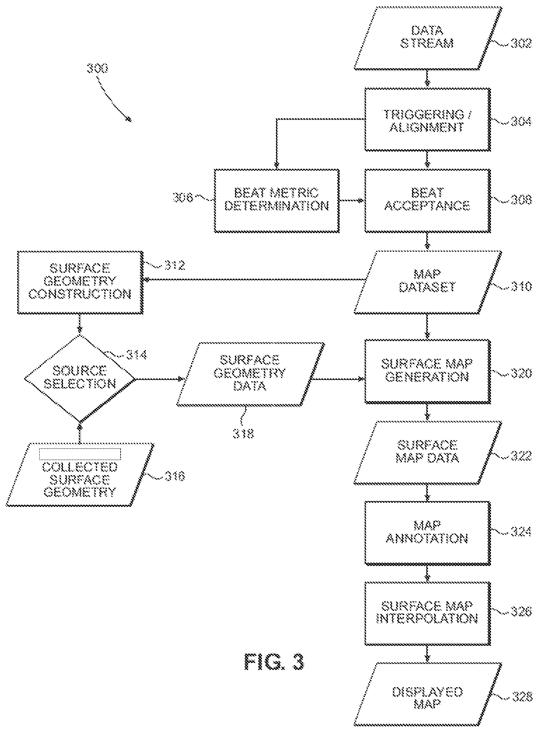

FIG. 3 is a flow diagram of an illustrative process 300 for automated anatomical mapping, in accordance with embodiments of the present disclosure. Aspects of embodiments of the illustrative process 300 may be performed, for example, by a processing unit (e.g., the processing unit 120 depicted in FIG. 1, and/or the processing unit 200 depicted in FIG. 2). A data stream 302 containing multiple signals is first input into the system (e.g., the cardiac mapping system 100 depicted in FIG. 1). During the automated electro-anatomical mapping process, the data stream 302 provides a collection of physiological and non-physiological signals that serve as inputs to the mapping process. The signals may be collected directly by the mapping system, and/or obtained from another system using an analog or digital interface. The data stream 302 may include signals such as unipolar and/or bipolar intracardiac electrograms (EGMs), surface electrocardiograms (ECGs), electrode location information originating from one or more of a variety of methodologies (magnetic, impedance, ultrasound, real time MRI, etc.), tissue proximity information, catheter force and/or contact information obtained from one or more of a variety of methodologies (force spring sensing, piezo-electric sensing, optical sensing etc.), catheter tip and/or tissue temperature, acoustic information, catheter electrical coupling information, catheter deployment shape information, electrode properties, respiration phase, blood pressure, other physiological information, and/or the like.

For the generation of specific types of maps, one or more signals may be used as one or more references, during a triggering/alignment process 304, to trigger and align the data stream 302 relative to the cardiac, other biological cycle and/or an asynchronous system clock resulting in beat datasets. Additionally, for each incoming beat dataset, a number of beat metrics are computed during a beat metric determination process 306. Beat metrics may be computed using information from a single signal, spanning multiple signals within the same beat and/or from signals spanning multiple beats. The beat metrics provide multiple types of information on the quality of the specific beat dataset and/or likelihood that the beat data is good for inclusion in the map dataset. A beat acceptance process 308 aggregates the criteria and determines which beat datasets will make up the map dataset 310. The map dataset 310 may be stored in association with a 3D grid that is dynamically generated during data acquisition.

Surface geometry data 318 may be generated concurrently during the same data acquisition process using identical and/or different triggering and/or beat acceptance metrics employing a surface geometry construction process 312. This process constructs surface geometry using data such as electrode locations and catheter shape contained in the data stream. Additionally, or alternatively, previously or concurrently collected surface geometry 316 may be used as an input to surface geometry data 318. Such geometry may have been collected previously in the same procedure using a different map dataset, and/or using a different modality such as CT, MRI, ultrasound, rotational angiography, and/or the like, and registered to the catheter locating system. The system performs a source selection process 314, in which it selects the source of the surface geometry data and provides surface geometry data 318 to a surface map generation process 320. The surface map generation process 320 is employed to generate surface map data 322 from the map dataset 310 and surface geometry data 318.

The surface geometry construction algorithm generates the anatomical surface on which the electroanatomical map is displayed. Surface geometry can be constructed, for example, using aspects of a system as described U.S. Patent 8,103,338, entitled "Impedance Based Anatomy Generation"; and/or U.S. Patent 8,948,837, entitled "Electroanatomical Mapping". Additionally, or alternatively, an anatomical shell can be constructed by the processing unit by fitting a surface on electrode locations that are determined either by the user or automatically to be on the surface of the chamber. In addition, a surface can be fit on the outermost electrode and/or catheter locations within the chamber.

As described, the map dataset 310 from which the surface is constructed can employ identical or different beat acceptance criteria from those used for electrical and other types of maps. The map dataset 310 for surface geometry construction can be collected concurrently with electrical data or separately. Surface geometry can be represented as a mesh containing a collection of vertices (points) and the connectivity between them (e.g. triangles). Alternatively, surface geometry can be represented by different functions such as higher order meshes, non-uniform rational basis splines (NURBS), and/or curvilinear shapes.

The generation process 320 generates surface map data 322. The surface map data 322 may provide information on cardiac electrical excitation, cardiac motion, tissue proximity information, tissue impedance information, force information, and/or any other collected information desirable to the clinician. The combination of map dataset 310 and surface geometry data 318 allows for surface map generation. The surface map is a collection of values or waveforms (e.g., EGMs) on the surface of the chamber of interest, whereas the map dataset can contain data that is not on the cardiac surface. One approach for processing the map dataset 310 and surface geometry data 318 to obtain a surface map dataset 322 is described in US 7,515,954, entitled "NON-CONTACT CARDIAC MAPPING, INCLUDING MOVING CATHETER AND MULTI-BEAT INTEGRATION" and filed June 13, 2006.

Alternatively, or in combination with the method above, an algorithm that applies acceptance criteria to individual electrodes can be employed. For example, electrode locations exceeding a set distance (e.g., 3mm) from surface geometry can be rejected. Another algorithm can incorporate tissue proximity information using impedance for inclusion in the surface map data. In this case only electrode location whose proximity value is less than 3mm might be included. Additional metrics of the underlying data can also be used for this purpose. For example, EGM properties similar to beat metrics can be assessed on a per electrode basis. In this case metrics such as far field overlap and/or EGM consistency can be used. It should be understood that variations on the method to project points from the map dataset 310 to the surface and/or to select appropriate points can exist.

Once obtained, the surface map data 322 may be further processed to annotate desired characteristics from the underlying data, a process defined as surface map annotation 324. Once data is collected into surface map data 322, characteristics relating to the collected data may be automatically presented to the user. These characteristics can be automatically determined and applied to the data by the computer system and are referred to herein as annotations. Exemplary annotations include activation time, the presence of double activation or fractionation, voltage amplitude, spectral content, average duty cycles, and/or the like. Due to the abundance of data available in automated mapping (e.g., mapping completed by the computer system with minimal human input related to the incoming data), it is not practical for the operator to review and annotate data manually. However, user input can be a valuable addition to the data, and so when user input is provided it is necessary for the computer system to automatically propagate and apply it to more than one data point at a time.

It may be possible to use the computer system to automatically annotate average duty cycles and other characteristics of individual or aggregated EGMs. The calculation and determination of average duty cycles and other characteristics is described in details in the present disclosure herein. Once computed, the annotations may be displayed superimposed on chamber geometry. In some embodiments, a gap-filling surface map interpolation may be employed 326. For example, in some embodiments, a gap-filling interpolation may be employed where a distance between a point on the surface to a measured EGM exceeds a threshold, as this may indicate, for example, that grid-based interpolation, as described herein, may not be as effective in that situation. Displayed maps 328 can be computed and displayed separately, and/or overlaid on top of each other.

The illustrative process 300 shown in FIG. 3 is not intended to suggest any limitation as to the scope of use or functionality of embodiments of the present disclosure. Neither should the illustrative process 300 be interpreted as having any dependency or requirement related to any single component or combination of components illustrated therein. Additionally, any one or more of the components depicted in FIG. 3 may be, for example, integrated with various ones of the other components depicted therein (and/or components not illustrated), all of which are considered to be within the ambit of the present disclosure.

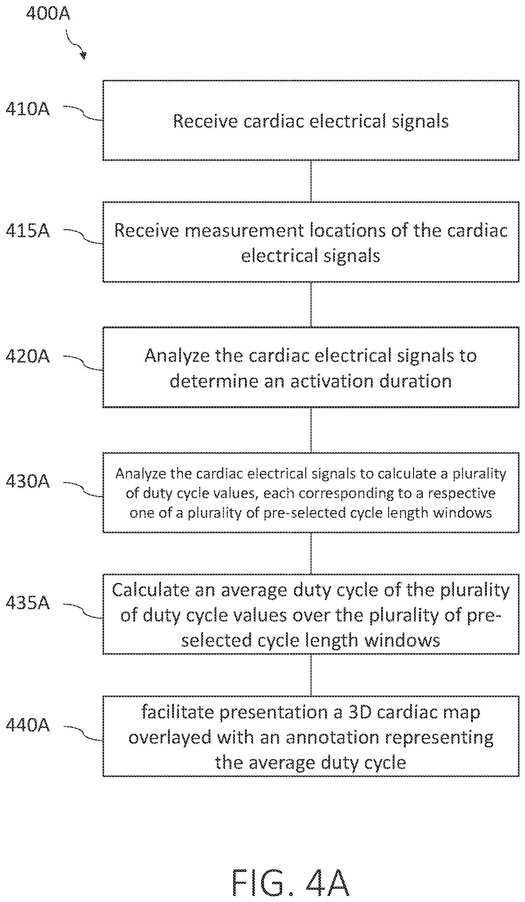

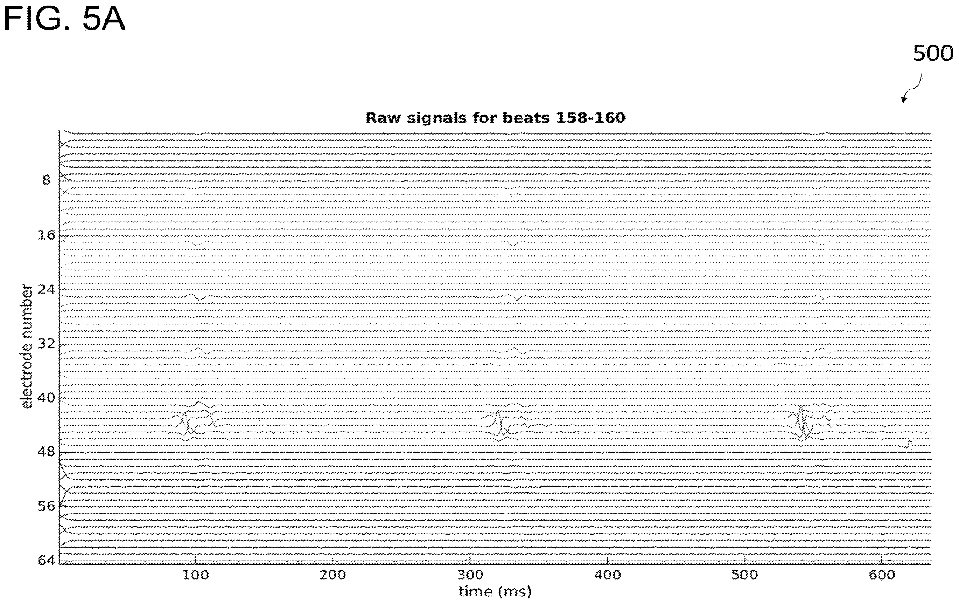

FIG. 4A is an example flow diagram depicting an illustrative method 400A of processing cardiac electrical signals and generated activation waveform, in accordance with some embodiments of the present disclosure. Aspects of embodiments of the method 400A may be performed, for example, by an electrophysiological system or a processing unit (e.g., the processing unit 120 depicted in FIG. 1, and/or the processing unit 200 depicted in FIG. 2). One or more steps of method 400A are optional and/or can be modified by one or more steps of other embodiments described herein. Additionally, one or more steps of other embodiments described herein may be added to the method 400A. First, the electrophysiological system receives one or more cardiac electrical signals (410A), collected from one or more electrodes disposed within a cardiac chamber, where the cardiac electrical signals are acquired over a cardiac beat. In some cases, the cardiac electrical signals comprise intra-cardiac electrograms (EGMs). FIG. 5A depicts an exemplary graphical representation 500 illustrating electrical signals (in this case, EGMs) received from a mapping catheter, each representing a magnitude of a depolarization sequence of a heart during a predetermined time period. In this example, EGMs of a mapping catheter having 64 electrodes are shown. Each waveform may represent unipolar signals received from an electrode of the mapping catheter. In some cases, each waveform, representing a cardiac electric signal, may present multipolar (e.g., bipolar, tripolar) signals received from electrodes.

The system may also receive an indication of a measurement location corresponding to each of the cardiac electrical signals (415A). In some embodiments, the system may analyze the one or more cardiac electrical signals to determine an activation duration (420A), associated with the cardiac beat. In embodiments, an activation duration may represent a length of an activation. That is, for example, a cardiac electrical signal (e.g., an EGM) may include a portion for which all of the amplitudes deviate beyond the signal baseline according to the specified criteria. The length of the time period corresponding to that portion of the cardiac electrical signal may be identified as an activation duration.

In some cases, the system analyzes the cardiac electrical signals to calculate a plurality of duty cycle values, where each calculated duty cycle value is corresponding to a respective one of a plurality of pre-selected cycle length windows (430A). In some cases, each duty cycle value is calculated by dividing the activation duration by a respective one of the plurality of pre-selected cycle length windows.

The system further calculates an average of the calculated duty cycle values over the plurality of pre-selected cycle length windows (435A). In embodiments, the calculated average duty cycle represents a probability that cardiac tissue corresponding to the measurement locations define a micro-reentry site. In some cases, the plurality of pre-selected cycle length windows fall within a range of from 140 milliseconds to 2000 milliseconds.

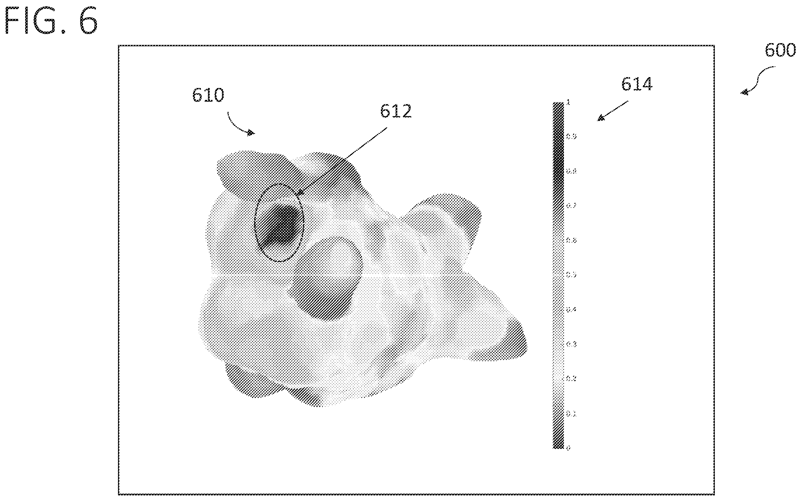

In embodiments, the system facilitates presentation, on a display device, a three-dimensional electroanatomical map overlayed with an annotation representing the calculated average duty cycle (440A), based on the measurement locations for the cardiac electrical signals associated therewith. In some cases, the system determines a plurality of average duty cycles at a plurality of sites. In some cases, the system comprises a display device (e.g., 170 of FIG. 1) operatively connected to the processing unit (e.g., 120 of FIG. 1, 200 of FIG. 2) and is configured to display the three-dimensional anatomical map overlayed with annotations. FIG. 6 depicts an illustrative example of an anatomical map with annotations representing average duty cycle values. In some cases, the values of the average duty cycle are represented by colors. In some cases, the values of the average duty cycle are represented by gray-scale values.

In some cases, the system is configured to calculate an average duty cycle value for each cardiac electrical signal. In some embodiments, the system is configured to aggregate a plurality of cardiac electrical signals having associated measurement locations within a specified region, and to calculate the average duty cycle value for the aggregated cardiac electrical signals. In some cases, the specified region has a predetermined geometry shape and size.

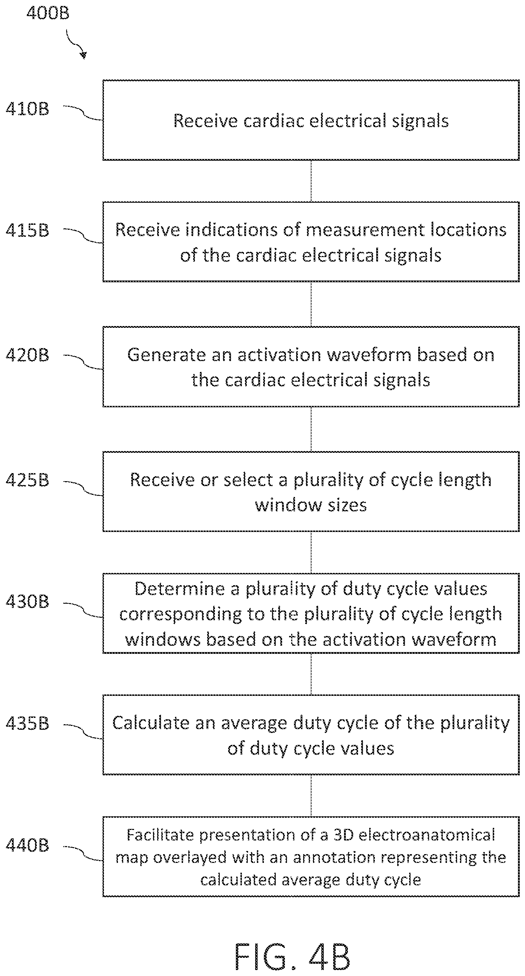

FIG. 4B is an example flow diagram depicting an illustrative method 4008 of processing cardiac electrical signals and generated activation waveform, in accordance with some embodiments of the present disclosure. Aspects of embodiments of the method 400B may be performed, for example, by an electrophysiological system or a processing unit (e.g., the processing unit 120 depicted in FIG. 1, and/or the processing unit 200 depicted in FIG. 2). One or more steps of method 400B are optional and/or can be modified by one or more steps of other embodiments described herein. Additionally, one or more steps of other embodiments described herein may be added to the method 400B. First, the electrophysiological system receives one or more cardiac electrical signals (4108), collected by one or more electrodes disposed in a cardiac chamber over a cardiac beat. In one embodiment, the cardiac electrical signals include inter-cardiac electrograms (EGMs). The system further receives indications of measurement locations of the cardiac electrical signals (4158). In some cases, each of the cardiac electrical signal has a corresponding measurement location.

In some embodiments, the electrophysiology system generates an activation waveform based on the cardiac electric signals (4208). The activation waveform includes a plurality of activation waveform values. In some cases, generating the activation waveform includes identifying deflections of the one or more cardiac electrical signals from a signal baseline. In some embodiments, each activation waveform value is associated with a probability of the identified deflection representing an activation. For example, in embodiments, the system may include determining a probability (e.g., a value between 0 and 1, inclusive) that a given sample point represents an activation, based on its relation to the signal baseline. In embodiments, other numerical scales may be used for assigning the probability such as, for example, values between 0 and 100, and/or the like. In embodiments, a likelihood (e.g., a probability) that a signal deflection represents an activation may be determined based on the deviation of that deflection from the signal baseline. For example, a deflection having a maximum amplitude that deviates from the signal baseline by at least a specified amount may be assigned a probability of 1, while a deflection having a maximum amplitude that deviates from the signal baseline by at most a specified amount may be assigned a probability of 0.

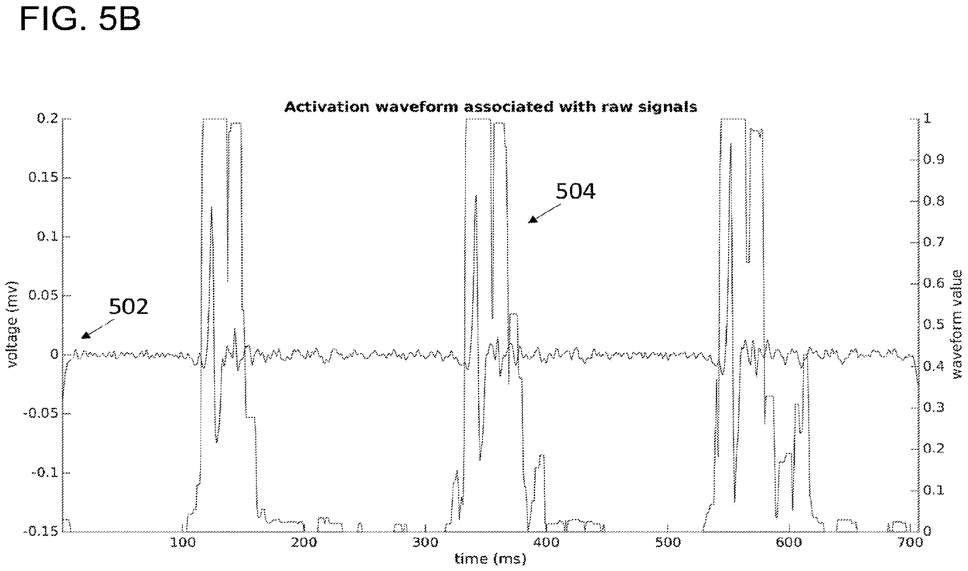

FIG. 5A depicts an exemplary graphical representation 500 illustrating electrical signals (in this case, EGMs) received from a mapping catheter, each representing a magnitude of a depolarization sequence of a heart during a predetermined time period. In this example, EGMs of a mapping catheter having 64 electrodes are shown. Each waveform may represent unipolar signals received from an electrode of the mapping catheter. In some cases, each waveform, representing a cardiac electric signal, may present multipolar (e.g., bipolar, tripolar) signals received from electrodes. FIG. 5B depicts a waveform of a raw cardiac electrical signal 502 and an activation waveform 504 corresponding to the cardiac electrical signal 502.

Referring back to FIG. 4B, in some embodiments, the electrophysiology system receives or selects a plurality of cycle length windows (425B). In some cases, the system selects the plurality of cycle length windows be a predetermined range, for example, a range of 120ms-2000ms. In some embodiments, the system receives an input associated with the plurality of cycle length windows, for example, by a user input (e.g., an input via a user interface such as a graphical user interface), a system input (e.g., system configuration), a software input (e.g., an input via an application programming interface, web service, etc.), and/or the like. In such embodiments, the system selects the plurality of cycle length windows based on the input. In one example, the system receives an input of a timing reference, such as 400ms, and set a range of 200ms-1600ms. In one embodiment, the plurality of cycle length windows are increased linearly (e.g., every 10 ms). In another embodiment, the plurality of cycle length windows are increased non-linearly.

In some embodiments, the system may analyze the one or more cardiac electrical signals to determine an activation duration, associated with the cardiac beat. In embodiments, an activation durations may represent a length of an activation. In some cases, the system determines the activation duration based on the activation waveform. In embodiments, the activation waveform may be represented along a time scale, in which case, the waveform may represent the activation duration. For example, the width of the deflection in the activation waveform may represent the duration of the corresponding activation.

According to the invention, the system determines a plurality of duty cycle values corresponding to the plurality of cycle length windows based on the activation waveform (4308). According to the invention, a duty cycle value for a cycle length window is determined as an average of activation waveform values in the cycle length window. In the example depicted in FIG. 5B, a cycle length window 506 of 250ms is selected and a duty cycle is determined as 0.26. In some embodiments, a duty cycle value is the activation duration divided by a respective one of the plurality of cycle length windows.

The system according to the invention further calculates an average of the plurality of duty cycles (435B). In some embodiments, the average is an arithmetic average. In some embodiments, the average is a weighted average. The electrophysiology system facilitates presentation of a 3D cardiac map overlayed with an annotation representing the calculated average of duty cycle values (440B). In embodiments, each annotation is at a corresponding measurement location. According to the invention, the calculated average duty cycle represents a probability that cardiac tissue corresponding to the measurement locations define a micro-reentry site. In some cases, a cardiac map is overlayed with annotations of micro-reentry probabilities, where the micro-reentry probabilities are represented by the calculated averages of duty cycle values. FIG. 6 depicts an exemplary electroanatomical map 600 that is annotated according to an embodiment. In this example, the electroanatomical map 600 is overlayed with annotations of micro-reentry probabilities 610. The legend of the micro-reentry probabilities is shown in 614. The 3D electroanatomical map can be gray scale image or a color image. In some cases, the values of average duty cycles, and/or micro-reentry probabilities, are represented by a color and/or a gray-scale value. In the example illustrated in FIG. 6, the region of 612 has high probabilities of micro-reentry ATs.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is defined by the appended claims.

## Claims

1. A system for processing cardiac information, the system comprising:
a processing unit configured to:
receive one or more cardiac electrical signals (502) from one or more electrodes disposed within a cardiac chamber, wherein the cardiac electrical signals are acquired over a cardiac beat and comprise intra-cardiac electrograms (EGMs);
receive an indication of a measurement location corresponding to each of the cardiac electrical signals;
analyze the one or more cardiac electrical signals to calculate a plurality of duty cycle values, each calculated duty cycle value corresponding to a respective one of a plurality of pre-selected cycle length windows (506), wherein the analysis of the one or more cardiac electric signals includes generating an activation waveform (504) therefrom, the activation waveform being based on deflections of the analyzed one or more cardiac electrical signals from a signal baseline, the activation waveform comprising values indicative of probabilities that the deflections represent activations of cardiac tissue, wherein the processing unit is configured to calculate the plurality of duty cycle values by calculating an average activation waveform value over each of the plurality of pre-selected cycle length windows;
calculate an average duty cycle of the calculated duty cycle values over the plurality of pre-selected cycle length windows, wherein the calculated average duty cycle represents a probability that cardiac tissue corresponding to the measurement locations define a micro-reentry site; and
facilitate presentation, on a display device, a three-dimensional electroanatomical map overlayed with an annotation representing the calculated average duty cycle based on the measurement locations for the cardiac electrical signals associated therewith.

2. The system of claim 1, wherein the plurality of pre-selected cycle length windows fall within a range of from 140 milliseconds to 2000 milliseconds.

3. The system of any of claims 1-2, further comprising a display device operatively connected to the processing unit and configured to display the three-dimensional anatomical map overlayed with the annotations.

4. The system of any of claims 1-3, wherein the processing unit is configured to calculate the average duty cycle value for each cardiac electrical signal.

5. The system of any of claims 1-3, wherein the processing unit is configured to aggregate a plurality of cardiac electrical signals having associated measurement locations within a specified region, and to calculate the average duty cycle value for the aggregated cardiac electrical signals.

6. A method of processing cardiac information, the method comprising:
receiving one or more cardiac electrical signals (502) acquired over a cardiac beat;
analyzing the one or more cardiac electrical signals and calculating a plurality of duty cycle values, each calculated duty cycle value corresponding to a respective one of a plurality of pre-selected cycle length windows (506), wherein the analyzing the one or more cardiac electrical signals includes generating an activation waveform (504) therefrom, the activation waveform being based on deflections of the one or more analyzed cardiac electrical signals from a signal baseline, the activation waveform comprising values indicative of probabilities that the deflections represent activations of cardiac tissue, wherein calculating the plurality of duty cycle values includes calculating an average activation waveform value over each of the plurality of pre-selected cycle length windows;
calculating an average of the calculated duty cycle values over the plurality of pre-selected cycle length windows, wherein the calculated average duty cycle represents a probability that cardiac tissue corresponding to the measurement locations define a micro-reentry site; and
displaying, on a display device, a three-dimensional anatomical map overlayed with an annotation representing the calculated average duty cycle.

7. The method of claim 6, wherein the plurality of pre-selected cycle length windows fall within a range of from 140 milliseconds to 2000 milliseconds.

8. The method of any one of claims 6-7, wherein the average duty cycle value is calculated for each cardiac electrical signal.

9. The method of any of claims 6-7, further including aggregating a plurality of cardiac electrical signals having associated measurement locations within a specified region, and calculating the average duty cycle value for the aggregated cardiac electrical signals.

## Patentansprüche

1. System zur Verarbeitung von kardiologischen Informationen, wobei das System umfasst:
eine Verarbeitungseinheit, die eingerichtet zum:
Empfangen eines oder mehrerer kardialer elektrischer Signale (502) von einer oder mehreren in einer Herzkammer angeordneten Elektroden empfangen, wobei die kardialen elektrischen Signale über einen Herzschlag erfasst werden und intrakardiale Elektrogramme (EGMs) umfassen;
Empfangen einer Angabe eines Messortes, der jedem der kardialen elektrischen Signale entspricht;
Analysieren des einen oder der mehreren kardialen elektrischen Signale, um eine Vielzahl von Arbeitszykluswerten zu berechnen, wobei jeder berechnete Arbeitszykluswert einem entsprechenden aus einer Vielzahl von vorgewählten Zykluslängenfenstern (506) entspricht, wobei die Analyse des einen oder der mehreren kardialen elektrischen Signale das Erzeugen einer Aktivierungswellenform (504) daraus umfasst, wobei die Aktivierungswellenform auf Ablenkungen des einen oder der mehreren analysierten kardialen elektrischen Signale von einer Signalbasislinie basiert, wobei die Aktivierungswellenform Werte umfasst, die Wahrscheinlichkeiten anzeigen, dass die Ablenkungen Aktivierungen von Herzgewebe darstellen, wobei die Verarbeitungseinheit eingerichtet ist, die Vielzahl von Arbeitszykluswerten durch Berechnen eines durchschnittlichen Aktivierungswellenformwertes über jedes der Vielzahl von vorgewählten Zykluslängenfenstern zu berechnen;
Berechnen eines durchschnittlichen Arbeitszyklusses der berechneten Arbeitszykluswerten über die Vielzahl von vorgewählten Zykluslängenfenstern, wobei das berechnete durchschnittliche Tastverhältnis eine Wahrscheinlichkeit darstellt, dass Herzgewebe, das den Messorten entspricht, eine Mikro-Wiedereintrittsstelle definiert; und
Erleichtern der Darstellung einer dreidimensionalen elektroanatomischen Karte auf einer Anzeigevorrichtung, die mit einer Anmerkung überlagert ist, die das berechnete durchschnittliche Arbeitszyklusses auf der Grundlage der Messorte für die damit verbundenen kardialen elektrischen Signale darstellt.

2. System nach Anspruch 1, wobei die Vielzahl von vorgewählten Zykluslängenfenster in einen Bereich von 140 Millisekunden bis 2000 Millisekunden fallen.

3. System nach einem der Ansprüche 1 bis 2, ferner umfassend eine Anzeigevorrichtung, die funktionsmäßig mit der Verarbeitungseinheit verbunden und eingerichtet ist, die dreidimensionale anatomische Karte überlagert mit den Anmerkungen anzuzeigen.

4. System nach einem der Ansprüche 1 bis 3, wobei die Verarbeitungseinheit eingerichtet ist, den durchschnittlichen Arbeitszykluswert für jedes kardiale elektrische Signal zu berechnen.

5. System nach einem der Ansprüche 1 bis 3, wobei die Verarbeitungseinheit eingerichtet ist, eine Vielzahl von kardialen elektrischen Signalen mit zugehörigen Messorten innerhalb eines bestimmten Bereichs zusammenzufassen und den durchschnittlichen Arbeitszykluswert für die zusammengefassten kardialen elektrischen Signale zu berechnen.

6. Verfahren zur Verarbeitung von kardiologischen Informationen, wobei das Verfahren umfasst:
Empfangen eines oder mehrerer kardialer elektrischer Signale (502), die während eines Herzschlags erfasst wurden
Analysieren des einen oder der mehreren kardialen elektrischen Signale, um eine Vielzahl von Arbeitszykluswerten zu berechnen, wobei jeder berechnete Arbeitszykluswert einem entsprechenden aus einer Vielzahl von vorgewählten Zykluslängenfenstern (506) entspricht, wobei die Analyse des einen oder der mehreren kardialen elektrischen Signale das Erzeugen einer Aktivierungswellenform (504) daraus umfasst, wobei die Aktivierungswellenform auf Ablenkungen des einen oder der mehreren analysierten kardialen elektrischen Signale von einer Signalbasislinie basiert, wobei die Aktivierungswellenform Werte umfasst, die Wahrscheinlichkeiten anzeigen, dass die Ablenkungen Aktivierungen von Herzgewebe darstellen, wobei Berechnen der Vielzahl von Arbeitszykluswerten Berechnen eines durchschnittlichen Aktivierungswellenformwertes über jedes der Vielzahl von vorgewählten Zykluslängenfenstern umfasst;
Berechnen eines durchschnittlichen Arbeitszyklusses der berechneten Arbeitszykluswerten über die Vielzahl von vorgewählten Zykluslängenfenstern, wobei das berechnete durchschnittliche Tastverhältnis eine Wahrscheinlichkeit darstellt, dass Herzgewebe, das den Messorten entspricht, eine Mikro-Wiedereintrittsstelle definiert; und
Anzeigen einer dreidimensionalen anatomischen Karte auf einer Anzeigevorrichtung, die mit einer Anmerkung überlagert ist, die den berechneten durchschnittlichen Arbeitszyklus darstellt.

7. Verfahren nach Anspruch 6, wobei die Vielzahl der vorgewählten Zykluslängenfenster in einen Bereich von 140 Millisekunden bis 2000 Millisekunden fällt.

8. Verfahren nach einem der Ansprüche 6 bis 7, wobei der durchschnittliche Arbeitszykluswert für jedes kardiale elektrische Signal berechnet wird.

9. Verfahren nach einem der Ansprüche 6 bis 7, ferner umfassend das Aggregieren einer Vielzahl von kardialen elektrischen Signalen mit zugehörigen Messorten innerhalb eines bestimmten Bereichs und das Berechnen des durchschnittlichen Arbeitszykluswertes für die aggregierten kardialen elektrischen Signale.

## Revendications

1. Système pour traiter une information cardiaque, le système comprenant :
une unité de traitement configurée pour :
recevoir un signal électrique cardiaque ou plusieurs signaux électriques cardiaques (502) en provenance d'une ou de plusieurs électrodes qui est/sont disposée(s) à l'intérieur d'une chambre cardiaque, dans lequel les signaux électriques cardiaques sont acquis sur un battement cardiaque et comprennent des électrogrammes (EGM) intracardiaques ;
recevoir une indication d'une localisation de mesure qui correspond à chacun des signaux électriques cardiaques ;
analyser les un ou plusieurs signaux électriques cardiaques afin de calculer une pluralité de valeurs de rapport cyclique, chaque valeur de rapport cyclique calculée correspondant à une fenêtre respective d'une pluralité de fenêtres de longueur de cycle présélectionnées (506), dans lequel l'analyse des un ou plusieurs signaux électriques cardiaques inclut la génération d'une forme d'onde d'activation (504) à partir de ceux-ci, la forme d'onde d'activation étant basée sur des déviations des un ou plusieurs signaux électriques cardiaques analysés par rapport à une ligne de base de signal, la forme d'onde d'activation comprenant des valeurs indicatives de probabilités que les déviations représentent des activations de tissu cardiaque, dans lequel l'unité de traitement est configurée pour calculer la pluralité de valeurs de rapport cyclique en calculant une valeur de forme d'onde d'activation moyenne sur chacune de la pluralité de fenêtres de longueur de cycle présélectionnées ;
calculer un rapport cyclique moyen des valeurs de rapport cyclique calculées sur la pluralité de fenêtres de longueur de cycle présélectionnées, dans lequel le rapport cyclique moyen calculé représente une probabilité qu'un tissu cardiaque qui correspond aux localisations de mesure définisse un site de micro-réentrée ; et
faciliter la présentation, sur un dispositif d'affichage, d'une carte électro-anatomique tridimensionnelle qui comporte, en superposition, une annotation qui représente le rapport cyclique moyen calculé sur la base des localisations de mesure pour les signaux électriques cardiaques associés afférents.

2. Système selon la revendication 1, dans lequel les fenêtres de la pluralité de fenêtres de longueur de cycle présélectionnées se situent à l'intérieur d'une plage de 140 millisecondes à 2 000 millisecondes.

3. Système selon l'une quelconque des revendications 1 et 2, comprenant en outre un dispositif d'affichage connecté de manière opérationnelle à l'unité de traitement et configuré pour afficher la carte anatomique tridimensionnelle qui comporte, en superposition, les annotations.

4. Système selon l'une quelconque des revendications 1 à 3, dans lequel l'unité de traitement est configurée pour calculer la valeur de rapport cyclique moyen pour chaque signal électrique cardiaque.

5. Système selon l'une quelconque des revendications 1 à 3, dans lequel l'unité de traitement est configurée pour agréger une pluralité de signaux électriques cardiaques présentant des localisations de mesure associées à l'intérieur d'une région spécifiée et pour calculer la valeur de rapport cyclique moyen pour les signaux électriques cardiaques agrégés.

6. Procédé de traitement d'une information cardiaque, le procédé comprenant :
la réception d'un signal électrique cardiaque ou de plusieurs signaux électriques cardiaques (502) qui est/sont acquis sur un battement cardiaque ;
l'analyse des un ou plusieurs signaux électriques cardiaques et le calcul d'une pluralité de valeurs de rapport cyclique, chaque valeur de rapport cyclique calculée correspondant à une fenêtre respective d'une pluralité de fenêtres de longueur de cycle présélectionnées (506), dans lequel l'analyse des un ou plusieurs signaux électriques cardiaques inclut la génération d'une forme d'onde d'activation (504) à partir de ceux-ci, la forme d'onde d'activation étant basée sur des déviations des un ou plusieurs signaux électriques cardiaques analysés par rapport à une ligne de base de signal, la forme d'onde d'activation comprenant des valeurs indicatives de probabilités que les déviations représentent des activations de tissu cardiaque, dans lequel le calcul de la pluralité de valeurs de rapport cyclique inclut le calcul d'une valeur de forme d'onde d'activation moyenne sur chacune de la pluralité de fenêtres de longueur de cycle présélectionnées ;
le calcul d'une moyenne des valeurs de rapport cyclique calculées sur la pluralité de fenêtres de longueur de cycle présélectionnées, dans lequel le rapport cyclique moyen calculé représente une probabilité qu'un tissu cardiaque qui correspond aux localisations de mesure définisse un site de micro-réentrée ; et
l'affichage, sur un dispositif d'affichage, d'une carte anatomique tridimensionnelle qui comporte, en superposition, une annotation qui représente le rapport cyclique moyen calculé.

7. Procédé selon la revendication 6, dans lequel les fenêtres de la pluralité de fenêtres de longueur de cycle présélectionnées se situent à l'intérieur d'une plage de 140 millisecondes à 2 000 millisecondes.

8. Procédé selon l'une quelconque des revendications 6 et 7, dans lequel la valeur de rapport cyclique moyen est calculée pour chaque signal électrique cardiaque.

9. Procédé selon l'une quelconque des revendications 6 et 7, incluant en outre l'agrégation d'une pluralité de signaux électriques cardiaques présentant des localisations de mesure associées à l'intérieur d'une région spécifiée et le calcul de la valeur de rapport cyclique moyen pour les signaux électriques cardiaques agrégés.
